# EUROPEAN PATENT APPLICATION

(11) **EP 3 225 226 A2**
(43) Date of publication of application: **04.10.2017**
(21) Application number: 17000087.1
(22) Date of filing: 18.01.2017
(51) Int. Cl.: A61H 31/00

(54) **CARDIOPULMONARY RESUSCITATION SUPPORT DEVICE**

(30) Priority: 30.03.2016 JP 2016069512
(71) Applicant: Sumitomo Riko Company Limited, Komaki-shi, Aichi 485-8550 (JP)
(72) Inventor: Kokubo, Yota, Komaki-shi Aichi, 485-8550 (JP); Hibino, Shingo, Komaki-shi Aichi, 485-8550 (JP); Sato, Kuninori, Komaki-shi Aichi, 485-8550 (JP); Minami, Kouichiro, Kawasaki-shi Kanagawa, 215-0036 (JP)
(74) Representative: Müller-Boré & Partner Patentanwälte PartG mbB

(57) **Abstract**

A cardiopulmonary resuscitation support device (10) for assisting a rescuer with cardiopulmonary resuscitation using chest compressions, including: a pressure detecting device (12) provided with a pressure sensor (16) including a pressure sensing part (40) which is configured to be overlapped on a chest, the pressure sensing part (40) having a sheet form and being capable of flexural deformation in a flexible manner; and a displacement detecting device (14) provided with a displacement sensor (64) to detect a displacement of a hand of the rescuer compressing the chest, the displacement detecting device (14) being configured to be retained by the hand of the rescuer at a part away from a chest compression surface.

## Description

### INCORPORATED BY REFERENCE

The disclosure of Japanese Patent Application No. 2016-069512 filed on March 30, 2016 including the specification, drawings and abstract is incorporated herein by reference in its entirety.

### BACKGROUND OF THE INVENTION

### 1. Field of the Invention

The present invention relates to a cardiopulmonary resuscitation support device that assists so as to perform chest compressions suitably with implementation or training of cardiopulmonary resuscitation using chest compressions for which a rescuer compresses the chest.

### 2. Description of the Related Art

From the past, cardiopulmonary resuscitation (CPR) using compressions to the chest has been known as one form of life support during cardiac arrest. With cardiopulmonary resuscitation, as is generally and broadly known, by a rescuer intermittently compressing the chest of a rescuee who is in cardiac arrest and artificially causing the heart to beat, the blood circulation and thus oxygen supply to the brain and the like attributable to this is maintained, and the heartbeat is prompted to restart.

Meanwhile, with cardiopulmonary resuscitation using chest compressions, it is thought to be important to have suitable levels of chest pressing depth and the number of compressions per unit of time during chest compression, to have sufficient chest release (recoil) in the intervals between chest compressions, and the ratio of the chest compression time (duty cycle) and the like. Through proper implementation of cardiopulmonary resuscitation, we can expect improved lifesaving rates, good prognoses and the like.

With regard to the chest compression and release, as disclosed in U.S. Patent No. US 7,220,235, it is suggested that a force sensor, or an accelerometer serving as a displacement sensor be arranged at a part to be compressed on the rescuee's chest, so as to determine good or bad based on the detection result obtained by the force sensor or the accelerometer. However, as described in US 7,220,235, with the force sensor, the chest compression depths in response to the input vary from one rescuee to another. Thus, if the compression depth is estimated based on the detected force, there is a risk of an error occurring. On the other hand, with the accelerometer, in addition to difficulty in setting the compression start position, in the cases where the surface that supports the rescuee during compression displaces in the up-down direction or the like, there is a problem such as deterioration in measurement accuracy of the chest compression depth.

US 7,220,235 also suggests using the force sensor and the accelerometer simultaneously so as to measure the chest compression and release. However, a hard accelerometer housing similar to a puck for ice hockey is arranged at a part to be compressed on the chest, and the rescuee's chest is compressed from above the accelerometer housing. Thus, it is difficult to mount and retain the cardiopulmonary resuscitation support device in a state of close contact on the chest surface of the rescuee, which is constituted by a complicated curving face with individual variation. This will tends to cause a problem of variability in detection precision due to wobbling of the accelerometer housing, and may cause troubles of deviation of the cardiopulmonary resuscitation support device on the chest, falling off of the device from the chest during the chest compressions, or the like. In addition, with chest compressions via the hard accelerometer housing, problems are likely to arise such as pain or injury to the rescuee's chest, or to the rescuer's hand.

Also, the rescuer who received normal cardiopulmonary resuscitation training is not used to doing compressions indirectly on the rescuee's chest via the hard accelerometer housing. Thus, using the cardiopulmonary resuscitation support device noted in US 7,220,235 would in fact have the risk of making it difficult to do suitable chest compressions. It is conceivable to use the cardiopulmonary resuscitation support device noted in US 7,220,235 during training, but in that case, conversely, in case of performing cardiopulmonary resuscitation in an emergency without the medium of the hard accelerometer housing that one is used to with training, it will be necessary to do direct chest compressions which are different from during training, and there is the risk of not being possible to suitably execute cardiopulmonary resuscitation.

### SUMMARY OF THE INVENTION

It is therefore one object of this invention to provide a cardiopulmonary resuscitation support device of novel structure which is able to reduce influence on measurement accuracy due to individual variation of the chest compression depth in response to the input, vibration of the surface that supports the rescuee, or the like, while being able to prevent foreign-body sensation or pain to the rescuer or rescuee at the compressed part.

The above and/or optional objects of this invention may be attained according to at least one of the following modes of the invention. The following modes and/or elements employed in each mode of the invention may be adopted at any possible optional combinations.

Specifically, a first mode of the present invention provides a cardiopulmonary resuscitation support device for assisting a rescuer with cardiopulmonary resuscitation using chest compressions, comprising: a pressure detecting device provided with a pressure sensor including a pressure sensing part which is configured to be overlapped on a chest, the pressure sensing part having a sheet form and being capable of flexural deformation in a flexible manner; and a displacement detecting device provided with a displacement sensor to detect a displacement of a hand of the rescuer compressing the chest, the displacement detecting device being configured to be retained by the hand of the rescuer at a part away from a chest compression surface.

With the cardiopulmonary resuscitation support device of construction according to the first mode, by using the pressure sensor and the displacement sensor, the chest compression depth can be measured with good accuracy.

Besides, since the displacement detecting device including the displacement sensor is retained by the rescuer's hand, the displacement of the rescuer's hand can be detected with better accuracy. Moreover, the displacement detecting device is retained by the rescuer's hand at a part away from the chest compression surface. This will prevent the rescuer's hand or the rescuee's chest from being injured or having pain due to the hard displacement detecting device.

A second mode of the present invention provides the cardiopulmonary resuscitation support device according to the first mode, further comprising: a base compression depth calculator to calculate a base compression depth for obtaining a chest compression depth based on the displacement detected by the displacement sensor; and a correlation estimator to set a correlation equation of a pressure detected by the pressure sensor and the chest compression depth based on the pressure detected by the pressure sensor and the base compression depth.

According to the second mode, it is possible to measure the chest compression depth based on the detection result obtained by the pressure sensor with high accuracy. Specifically, the chest compression depth with respect to the detection value of the pressure varies among individuals depending on the physique, sex, or the like of the rescuee. On the other hand, with regard to the base compression depth calculated based on the displacement of the rescuer's hand, which is detected on the stable surface, influence due to the individual variation of the rescuee is small, so that the base compression depth corresponds with the actual chest compression depth with good accuracy. Therefore, from the correlation equation of the base compression depth and the detection value of the pressure, the correlation estimator will derive the correlation between the detection value of the pressure and the actual chest compression depth, in other words, the correction coefficient of the detection value of the pressure according to the ease in displacement of the rescuee's chest, so as to make it possible to estimate the chest compression depth from the detection value of the pressure with good accuracy. By so doing, in measuring of the chest compression depth based on the pressure, errors in the measurement results due to individual variation of the rescuee can be reduced or avoided, so that high-accuracy measurements will be possible.

Additionally, by measuring the chest compression depth based on the pressure, even in the case where the surface on which the rescuee lies is unstable due to displacement, deformation, or the like, an accurate measurement is possible. Specifically, if the surface on which the rescuee lies displaces or deforms in the direction of chest compressions, the displacement sensor cannot discriminate between the displacement by movement of the rescuer's hand and the displacement by movement of the rescuee in detection. In this case, with the measurement of the chest compression depth based on the displacement of the rescuer's hand, the measurement accuracy may be deteriorated. On the other hand, the pressure sensor detects the pressure acted on the rescuee's chest regardless of movement of the rescuee in the direction of chest compressions. Thus, with the measurement of the chest compression depth based on the detection value of the pressure, effective measurements will be possible even in the case where the rescuee moves in the direction of chest compressions.

A third mode of the present invention provides the cardiopulmonary resuscitation support device according to the first or second mode, further comprising at least one display monitor to display detected information.

According to the third mode, the rescuer sees the detected information displayed on the display monitor, and the rescuer is able to grasp the points to be improved, the way to improve, or the like, for the chest compressions.

A fourth mode of the present invention provides the cardiopulmonary resuscitation support device according to the third mode, wherein the at least one display monitor comprises a plurality of display monitors positioned near the pressure detecting device, and each of the display monitors is configured to display the detected information.

According to the fourth mode, in the case where a plurality of rescuers perform rescue operations in cooperation with one another, the rescuers are able to perform rescue operations while checking the detected information displayed on the individual display monitors. Therefore, it is possible for the rescuers other than the chest compression performer to read the detected information and report it to the chest compression performer, to check fatigue of the chest compression performer based on the detected information and take turns performing the chest compressions, or the like.

A fifth mode of the present invention provides the cardiopulmonary resuscitation support device according to any one of the first through fourth modes, further comprising a wireless communicator capable of communication with an emergency center.

According to the fifth mode, communication with emergency centers such as a firehouse, an emergency hospital, security or the like through the wireless communicator makes it possible to request for an ambulance or dispatch of rescue cooperators as well as to receive instructions for a correct way to perform the chest compressions, or the like. Moreover, it is possible to receive guidance to a place where an AED (Automated External Defibrillator) is located (AED location) and which is the nearest from the site of the rescue operations, or it is possible for the emergency center to make contact with the AED location which is the nearest from the site of the rescue operations and to instruct to carry the AED to the said site, and the like. Furthermore, by transmitting the detected information on the chest compressions being performed to the emergency center, the operator etc. of the emergency center is able to give advice on the chest compressions based on the detected information, and in addition to that, more effective lifesaving activities will be possible based on the accumulation of the detected information.

A sixth mode of the present invention provides the cardiopulmonary resuscitation support device according to the fifth mode, further comprising a display monitor to display a communication state by the wireless communicator.

According to the sixth mode, the rescuer confirms the information on the communication state displayed on the display monitor, and the rescuer is readily able to grasp whether the communication with the emergency center is performed or not.

A seventh mode of the present invention provides the cardiopulmonary resuscitation support device according to the fifth or sixth mode, further comprising a communication instructor to automatically start the communication by the wireless communicator when a use of at least one of the displacement detecting device and the pressure detecting device is detected.

According to the seventh mode, the communication by the wireless communicator will be automatically started on using the cardiopulmonary resuscitation support device. Thus, for example, by automatically transmitting the request for an ambulance, information on the place where the rescue operations are performed, or the like to the emergency center, the chest compressions can be immediately started without spending time on notification, thereby improving lifesaving rates.

An eighth mode of the present invention provides the cardiopulmonary resuscitation support device according to any one of the fifth through seventh modes, wherein the displacement detecting device comprises a mobile phone, and the displacement detecting device includes the wireless communicator.

According to the eighth mode, the displacement detecting device comprises a mobile phone such as a smart phone or the like. Thus, even a citizen rescuer who happens to be at the site can perform the lifesaving activity by utilizing the mobile phone in hand. Moreover, the smart phone, which is one type of a mobile phone, is able to communicate with the emergency center by means of its communication (call) function, as well as to transmit the location information to the emergency center by means of GPS (Global Positioning System) or the like, thereby realizing effective lifesaving activity. In addition, the mobile phone, which is generally operatable by one hand, has a size that is easy to retain with the rescuer's hand, and is less likely to hinder the chest compressions.

A ninth mode of the present invention provides the cardiopulmonary resuscitation support device according to any one of the first through eighth modes, wherein the pressure sensor comprises a capacitance type pressure sensor having a structure in which a plurality of pressure detection parts are arranged two dimensionally, each pressure detection part comprising flexible electrodes placed in opposition and a flexible dielectric layer having electric insulation properties disposed between the electrodes, and the pressure sensor is configured to detect a pressure acting on the chest during the chest compressions based on an amount of change in electrostatic capacity of each pressure detection part generated by a change in distance between the electrodes when the pressure acts due to the chest compressions.

According to the ninth mode, the pressure sensor is provided with the flexible pressure sensing part constituted by the plurality of pressure detection parts each comprising the flexible electrodes and the dielectric layer. This will reduce sense of discomfort during the chest compressions and will not create a problem of pain, injury or the like to the rescuer's hand or the rescuee's chest during the chest compressions. Besides, by detecting the electrostatic capacity of the plurality of pressure detection parts arranged two dimensionally, it is possible to detect the pressure acting on the chest over the prescribed area, as well as to detect distribution of the pressure.

A tenth mode of the present invention provides the cardiopulmonary resuscitation support device according to any one of the first through ninth modes, wherein the displacement sensor of the displacement detecting device comprises an acceleration sensor.

According to the tenth mode, by performing second-order integration of the acceleration detected by the acceleration sensor, the displacement of the rescuer's hand can be obtained.

According to the present invention, it is possible to measure the chest compression depth with good accuracy by utilizing the pressure sensor and the displacement sensor, as well as to detect the displacement of the rescuer's hand with better accuracy by the displacement detecting device including the displacement sensor being retained by the rescuer's hand. Furthermore, since the displacement detecting device is retained by the rescuer's hand at the part away from the chest compression surface, the rescuer's hand or the rescuee's chest can be prevented from being injured or having pain due to the hard displacement detecting device.

### BRIEF DESCRIPTION OF THE DRAWINGS

The foregoing and/or other objects, features and advantages of the invention will become more apparent from the following description of a preferred embodiment with reference to the accompanying drawings in which like reference numerals designate like elements and wherein:
FIG. 1 is a front view schematically showing a usage of a cardiopulmonary resuscitation support device according to a first embodiment of the present invention;
FIG 2 is a top plane view schematically showing the usage of the cardiopulmonary resuscitation support device shown in FIG 1;
FIG 3 is a top plane view of the cardiopulmonary resuscitation support device shown in FIG 1;
FIG 4 is an exploded perspective view of a pressure sensor of the cardiopulmonary resuscitation support device shown in FIG 1;
FIG 5 is a schematic configuration diagram of a sensor controller of the cardiopulmonary resuscitation support device shown in FIG 1;
FIG 6 is a top plane view of a smart phone of the cardiopulmonary resuscitation support device shown in FIG. 1;
FIG 7 is a schematic configuration diagram of the smart phone shown in FIG. 6;
FIG 8 is a functional block diagram of the cardiopulmonary resuscitation support device shown in FIG. 1; and
FIG 9 is a view showing an emergency lifesaving care system using the cardiopulmonary resuscitation support device shown in FIG. 1.

### DETAILED DESCRIPTION OF PREFERRED EMBODIMENTS

Embodiments of the present invention will be described below in reference to the drawings.

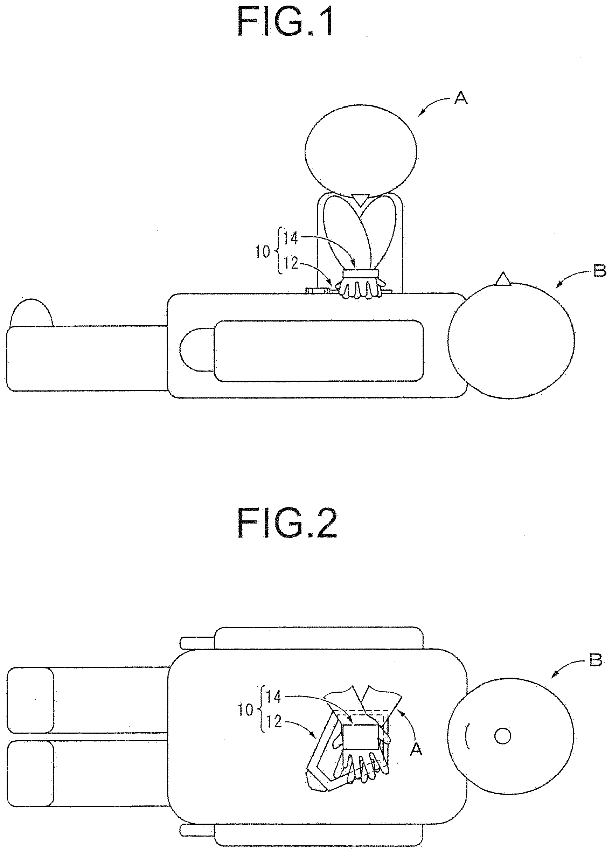

FIGS. 1 and 2 depict a usage of a cardiopulmonary resuscitation support device 10 according to a first embodiment of the present invention. The cardiopulmonary resuscitation support device 10 is a device for assisting cardiopulmonary resuscitation using chest compressions performed by a rescuer A on a rescuee B, and includes a pressure detecting device 12 to be overlapped on a chest of the rescuee B and a smart phone 14 serving as a displacement detecting device to be retained by a hand of the rescuer A. In the description hereinbelow, as a general rule, the up-down direction refers to the up-down direction in FIG 1, which coincides with the vertical up-down direction.

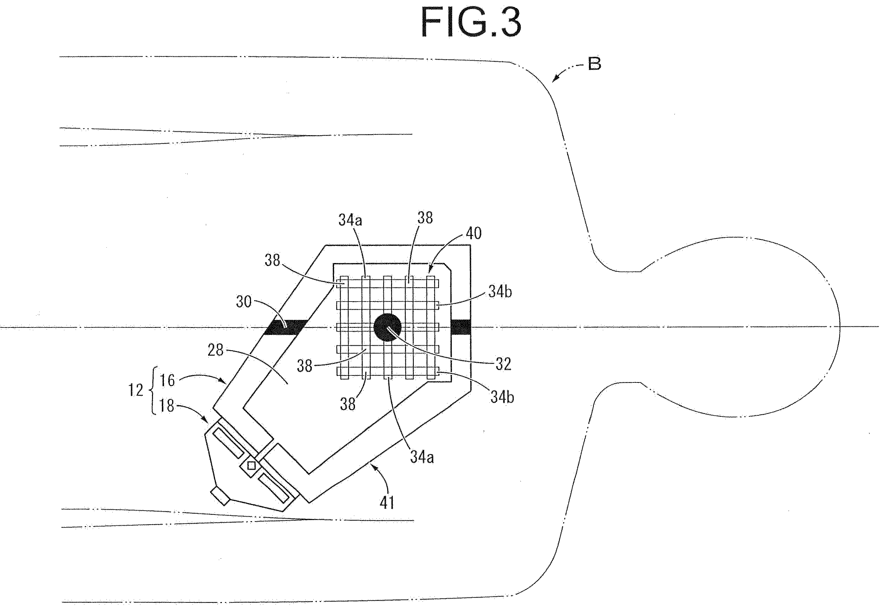

Described more specifically, as depicted in FIG. 3, the pressure detecting device 12 includes a pressure sensor 16 to detect the input by the chest compressions and a sensor controller 18 to process the detection results obtained by the pressure sensor 16 or the like.

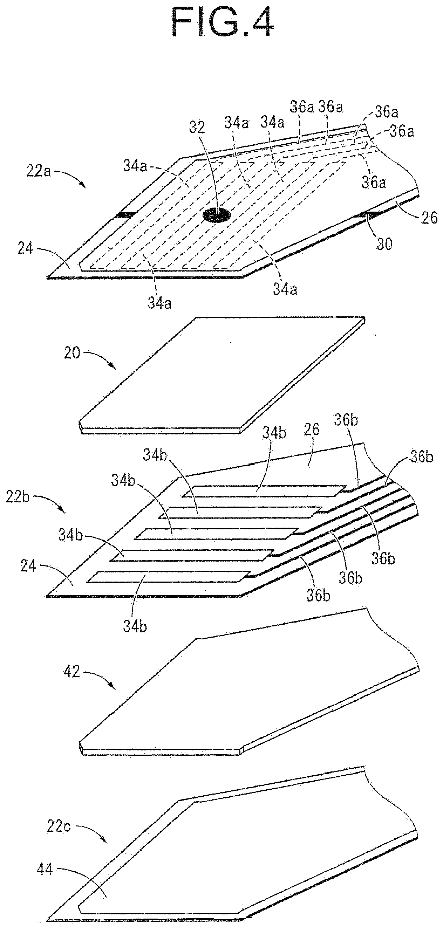

The pressure sensor 16 is a capacitance type pressure sensor that detects the pressure based on the change in electrostatic capacity, and as depicted in FIG 4, has a structure including a dielectric layer 20, an elastomer sheet 22a overlapped on one face of the dielectric layer 20, and an elastomer sheet 22b overlapped on the other face of the dielectric layer 20.

The dielectric layer 20 is formed using an elastomer having electric insulation properties such as rubber, resin or the like, has a plate shape or sheet shape, has elasticity or flexibility, can be deformed by expansion and contraction, and in particular can be easily deformed in the thickness direction. As the forming material of the dielectric layer 20, for example, it is preferable to use silicone rubber, acrylonitrile-butadiene copolymer rubber, acrylic rubber, epichlorohdyrin rubber, chlorosulfonated polyethylene, chlorinated polyethylene, urethane rubber, polyethylene resin, polypropylene resin, polyurethane resin, polystyrene resin, polyvinyl chloride-polyvinylidene chloride copolymer, ethylene-acetic acid copolymer or the like. Furthermore, the dielectric layer 20 can also be foam, and as long as the necessary dielectric constant and flexibility are ensured, that foam is not limited to being an item that exhibits a uniform phase with independent bubbles, but for example can also exhibit a non-uniform phase by continuous bubbles being formed, or the like. Also, the thickness and forming material of the dielectric layer 20 and the like are suitably set according to the relative dielectric constant and flexibility required in pressure detection parts 38 described later.

The elastomer sheet 22a/22b is formed using a thin-walled, sheet-shaped elastomer having electric insulation properties such as rubber, resin or the like, so as to be permitted flexural deformation in the thickness direction. As shown in FIGS. 3 and 4, the elastomer sheet 22a/22b of the present embodiment has a structure in which an electrode formation zone 24 and a wiring formation zone 26 are integrally formed, where the electrode formation zone 24 has a roughly square shape and constitutes a pressure sensing part 40 described later, and the wiring formation zone 26 extends from a circumferential portion of the electrode formation zone 24 in the direction of extension of a diagonal thereof and constitutes a wiring part 41 described later. Note that as the forming material of the elastomer sheet 22a/22b, whereas the material similar to that of the dielectric layer 20 can be used, it is desirable to use a non-foaming material in order to stably form electrodes 34a, 34b described later.

Moreover, on the upper surface of the upper elastomer sheet 22a, an upper noise guard 28 formed of a conductive material is provided. The upper noise guard 28 is provided so as to cover the formation zone of the electrodes 34a, 34b and wires 36a, 36b described later, and is configured to reduce influence on the detection results of the pressure (electrostatic capacity) due to a touch by the rescuer A's hand or the like.

Furthermore, the upper surface of the upper elastomer sheet 22a, a positioning line 30 is marked. The positioning line 30 is a straight line marked at the location which is equivalent to the lateral center of the rescuee B when the pressure sensor 16 is overlapped on the rescuee B's chest, and extends in the body axis direction of the rescuee B. Besides, the positioning line 30 is provided off the formation zone of the upper noise guard 28 so as to be segmented in the lengthwise direction by the upper noise guard 28. Note that the positioning line 30 may be drawn by printing or the like on the upper elastomer sheet 22a, or may be a concave groove or a convex ridge.

Additionally, on the upper surface of the upper elastomer sheet 22a, a center marker 32 is provided. The center marker 32 indicates the center to be compressed in the pressure sensing part 40 described later, and in the present embodiment, the center marker 32 is a circular mark printed on the upper surface of the upper noise guard 28. Note that the center marker 32 is located on the extension of the positioning line 30 provided off the upper noise guard 28.

The elastomer sheet 22a/22b includes electrodes 34a/34b in the electrode formation zone 24 on the surface overlapped on the dielectric layer 20. The electrodes 34a/34b are, for example, formed using a flexible conductive elastomer for which a conductive filler (e.g. carbon black, a metal powder such as silver powder or the like) is added to an elastomer such as rubber, resin or the like, and are easily deformable. As the elastomer which is the forming material of the electrodes 34a/34b, it is preferable to use, for example, silicone rubber, ethylene-propylene copolymer rubber, natural rubber, styrene-butadiene copolymer rubber, acrylonitrile-butadiene copolymer rubber, acrylic rubber, epichlorohydrin rubber, chlorosulfonated polyethylene, chlorinated polyethylene, urethane rubber, polyester resin, polyether urethane resin, polycarbonate urethane resin, vinyl chloride-vinyl acetate copolymer, phenol resin, acrylic resin, polyamide-imide resin, polyamide resin, nitrocellulose, modified celluloses or the like. Note that the elastomer sheet 22a/22b may be formed of the material similar to that of the electrodes 34a/34b.

Moreover, each electrode 34a/34b has a thin-walled, elongated band shape, and a plurality of the electrodes 34a/34b are arranged side by side in the electrode formation zone 24 on the elastomer sheet 22a/22b so as to be remote from one another by a prescribed distance in the short-side direction. Also, the electrodes 34a provided on the elastomer sheet 22a overlapped on one surface of the dielectric layer 20 and the electrodes 34b provided on the elastomer sheet 22b overlapped on the other surface of the dielectric layer 20 extend in the planar directions roughly orthogonal to each other.

Furthermore, wires 36a, 36b are connected to the respective electrodes 34a, 34b. The wires 36a/36b are formed on the surface of the wiring formation zone 26 of the elastomer sheet 22a/22b as a wiring pattern printed using, for example, conductive ink. The wires 36a connected to the electrodes 34a extend to the end of the wiring formation zone 26 of the elastomer sheet 22a, while the wires 36b connected the electrodes 34b extend to the end of the wiring formation zone 26 of the elastomer sheet 22b.

Then, the electrode formation zone 24 of the elastomer sheet 22a is overlapped on the upper surface of the dielectric layer 20, while the electrode formation zone 24 of the elastomer sheet 22b is overlapped on the lower surface of the dielectric layer 20. The portions of the elastomer sheets 22a, 22b which are off the dielectric layer 20 are overlapped and bonded to each other.

By so doing, the electrodes 34a formed in the electrode formation zone 24 of the elastomer sheet 22a and the electrodes 34b formed in the electrode formation zone 24 of the elastomer sheet 22b intersect and oppose each other at a plurality of locations, and the dielectric layer 20 is sandwiched between the electrodes 34a, 34b at those intersecting and opposing parts. At the opposing parts where the electrodes 34a, 34b intersect, capacitors are constituted so as to form the pressure detection parts 38 serving as capacitance type sensors which are able to detect input based on changes in the electrostatic capacity of the capacitor. It would be acceptable to provide only one pressure detection part 38, however, in the present embodiment, by arranging the electrodes 34a and the electrodes 34b intersecting at the plurality of locations, a plurality of pressure detection parts 38 are dispersed and provided two dimensionally. Also, with the plan view shown in FIG 3, the dielectric layer 20 is formed in a shape that is roughly the same or larger than the arrangement region of the electrodes 34a, 34b of the elastomer sheets 22a, 22b, and with all the pressure detection parts 38, the dielectric layer 20 is sandwiched between the electrodes 34a, 34b. In FIG 3, the electrodes 34a, 34b and the pressure detection parts 38 are illustrated with fine lines.

At the intersecting and opposing parts of these electrodes 34a, 34b (pressure detection parts 38), when the dielectric layer 20 is deformed by compression in the layering direction of the dielectric layer 20 and the elastomer sheets 22a, 22b, the opposing distance between the electrodes 34a, 34b becomes shorter, so that the electrostatic capacity of that part changes. Taking advantage of this, by using the sensor controller 18 comprising an electrical control device, it is possible to detect changes in the electrostatic capacity of each pressure detection part 38, which makes it possible to detect changes in the opposing distance between the electrodes 34a, 34b with each pressure detection part 38. This constitutes a pressure sensing part 40 which detects distribution of the pressure acting over the prescribed range and is capable of flexural deformation in a flexible manner. In this way, each intersecting part of the electrodes 34a, 34b (pressure detection part 38) can function as a capacitance type detection element (cell). With the pressure detection part 38, the changes in the electrostatic capacity that accompany changes in the opposing distance between the electrodes 34a, 34b are detected, and it is also possible to detect changes in the electrostatic capacity by changes in the substantial opposing surface area due to the expansion and contraction or relative displacement in the planar direction of the electrodes 34a, 34b. Specifically, during the chest compressions described later, not only are changes in the electrostatic capacity accompanying changes in the opposing distance detected, but it is also possible to detect changes in the electrostatic capacity due to relative displacement in the planar direction or expansion and contraction.

The surface area of the pressure sensing part 40 according to the present embodiment is the average surface area or greater of the chest compression part of a person's palm. Preferably, for example, it is the average surface area (e.g. about 900 mm²) or greater of the thenar (the part of the palm that rises up at the base part of the thumb) which is the substantial chest compression part of a person's palm, and this makes it possible to sufficiently deal with skew of the compression position. Also, more preferably, by having it be the average surface area or greater than the overall palm of a person, it is also possible to confirm whether or not pressing is occurring at a suitable part of the palm, or the like.

Moreover, by the wiring formation zones 26, 26 including the respective wires 36a, 36b being overlapped on each other, the wiring part 41 is formed. The wiring part 41 extends from a circumferential portion of the pressure sensing part 40 to the outside in the planar direction. With the pressure sensing part 40 set on the rescuee B's chest as will be described later, the wiring part 41 is provided so as to extend in the diagonal direction that inclines with respect to the body axis of the rescuee B. By so doing, the wiring part 41 is less likely to be an obstacle to the rescue operation by the rescuer A. In particular, by having the wiring part 41 extend to the rescuer A's leg side, even when a plurality of rescuers (the rescuer A who performs the chest compressions and the rescue cooperators who perform the other rescue operations) perform the rescue operations on the left-right sides and on the head part side of the rescuee B, the wiring part 41 can be prevented from being an obstacle.

Also, an insulator layer 42 is overlapped on the lower surface of the elastomer sheet 22b. The insulator layer 42 is formed using a soft synthetic resin, a rubber elastic body or the like having electric insulation properties in addition to flexibility and elasticity. The insulator layer 42 has a sheet form or plate form corresponding to the elastomer sheet 22b, and is overlapped on the opposite side to the dielectric layer 20 in relation to the elastomer sheet 22b. As the forming material of the insulator layer 42, it is preferable to use polyethylene, urethane rubber or the like.

Furthermore, a lower noise guard 44 provided on an elastomer sheet 22c is overlapped on the insulator layer 42. The lower noise guard 44 is formed in a thin film form using a conductive elastomer that is flexible and can expand and contract, the same as the electrodes 34a, 34b, and is overlapped on the opposite side to the elastomer sheet 22b in relation to the insulator layer 42. By so doing, the lower noise guard 44 is overlapped via the insulator layer 42 on the electrodes 34b and the wires 36b of the elastomer sheet 22b, and is electrically insulated by the insulator layer 42.

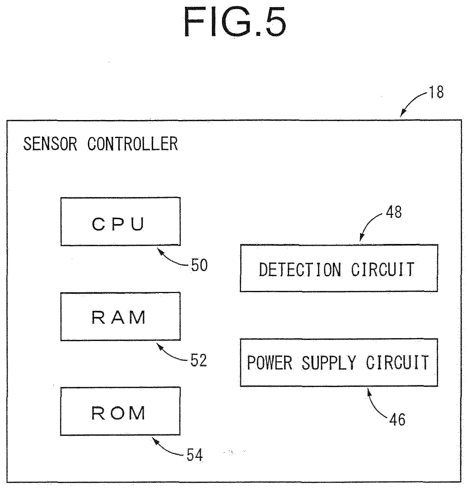

Besides, as depicted in FIG. 3, the sensor controller 18 is connected to the wiring part 41 of the pressure sensor 16. As shown in FIG. 5, the sensor controller 18 is equipped with, for example, a power supply circuit 46 for supplying operating voltage and a detection circuit 48 for detecting or obtaining electrostatic capacity, which are connected via the wires 36a, 36b to the respective electrodes 34a, 34b. The power supply circuit 46 selectively performs power feed to the plurality of electrodes 34a and the plurality of electrodes 34b, and under control by a central processing unit (CPU) 50, at each intersecting part at 25 locations constituting the capacitors, periodic waveform voltage is applied in scanning form as measurement voltage.

Also, the detection signals of the electrostatic capacity detected under this voltage action are detected in sequence by the detection circuit 48, and the detection values are stored in a RAM (random access memory) 52. Detection of electrostatic capacity by the detection circuit 48 is performed by obtaining the electrostatic capacity values using the impedance obtained from the current values, for example.

In addition, the characteristics data of the capacitors constituted by the intersecting parts of the electrodes 34a, 34b are stored in a ROM (read only memory) 54. Based on this characteristics data, using the CPU 50, it is also possible to obtain the compression force that is the external force applied to the intersecting parts of the electrodes 34a, 34b from the detection values of the electrostatic capacity obtained excluding the effect of wiring resistance.

Therefore, by respectively scanning the electrostatic capacity detected by the 25 pressure detection parts 38 constituted by the intersecting parts of the electrodes 34a, 34b constituting the capacitors, it is possible to detect the two dimensional distribution of electrostatic capacity values overall. It is also possible to obtain the two dimensional distribution of the compression force based on the electrostatic capacity detection value.

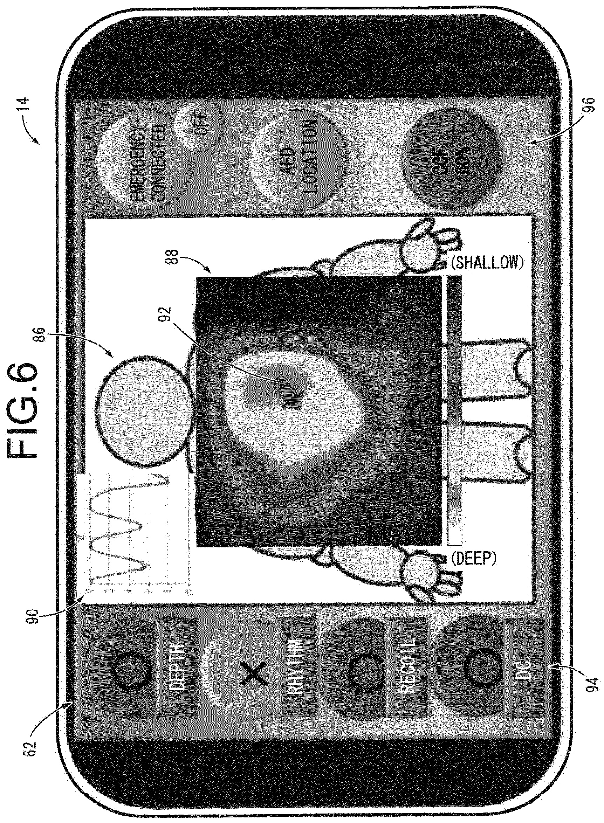

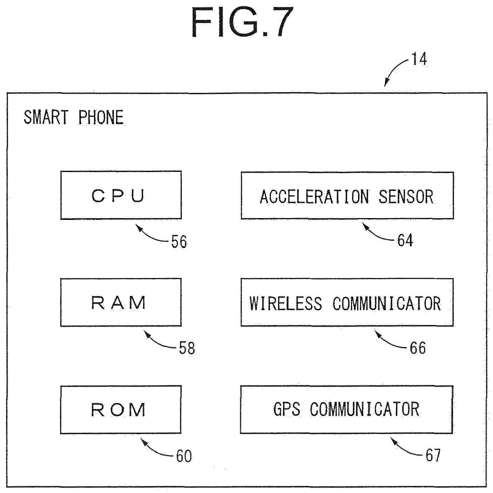

Meanwhile, the smart phone 14 is a mobile phone as shown in FIG 6. As shown in FIG. 7, the smart phone 14 has a hardware configuration including a central processing unit (CPU) 56, a RAM 58, a ROM 60, a display 62 serving as a display monitor, and has a call function, a data communication function, or the like which a typical smart phone has.

Moreover, the smart phone 14 includes an acceleration sensor 64 to detect acceleration which serves as a displacement sensor. The acceleration sensor 64 is, for example, a semiconductor acceleration sensor which is capacitance type, piezoresistance type or the like. Under control by the CPU 56, the acceleration detection signal is detected regularly or continuously, and then the detection value is stored in the RAM 58.

Also, the smart phone 14 includes a wireless communicator 66 that realizes voice communication and data communication with other terminals. This makes it possible for the smart phone 14 to, for example, call an emergency center 98 described later, transmit the results measured by the pressure detecting device 12 or the smart phone 14 to the emergency center 98, or the like.

The smart phone 14 further includes a GPS communicator 67 for receiving location information from an artificial satellite via an earth station. Then, the location information obtained by the GPS communicator 67 can be transmitted to the emergency center 98 described later by the wireless communicator 66.

Besides, the smart phone 14 and the pressure detecting device 12 are connected to each other, so that a signal based on the acceleration detection value (base compression depth signal) transmitted from the smart phone 14 can be received by the pressure detecting device 12, while a signal based on the pressure detection value (chest compression depth signal) transmitted from the pressure detecting device 12 can be received by the smart phone 14.

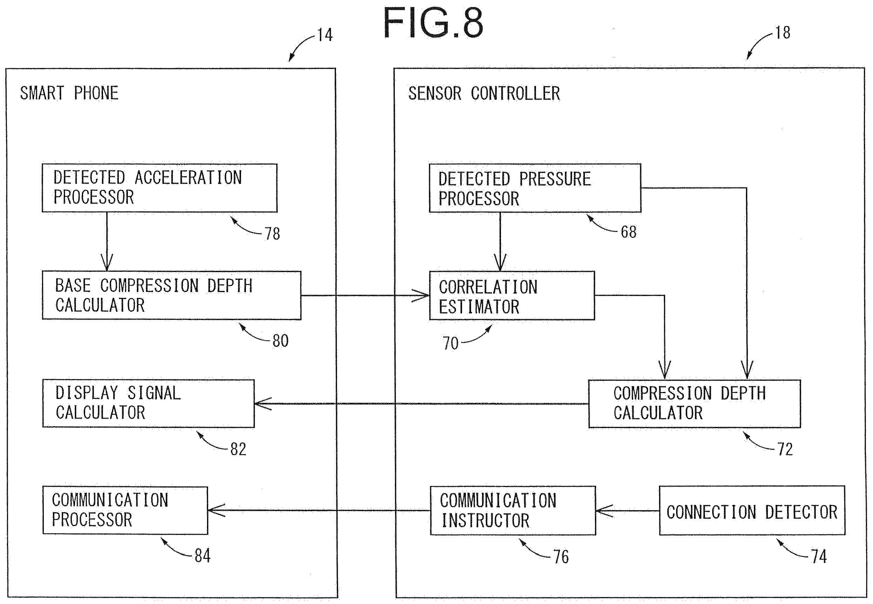

FIG. 8 shows a functional block diagram of the cardiopulmonary resuscitation support device 10. Specifically, the sensor controller 18 of the pressure detecting device 12 includes a detected pressure processor 68, a correlation estimator 70, a compression depth calculator 72, a connection detector 74, and a communication instructor 76. Meanwhile, the smart phone 14 includes a detected acceleration processor 78, a base compression depth calculator 80, a display signal calculator 82, and a communication processor 84. Hereinbelow, there will be described the function of each component of the cardiopulmonary resuscitation support device 10 during the chest compressions.

When the chest compressions start, the detected pressure processor 68 detects the pressure acting on the rescuee B's chest as the change in electrostatic capacity and generates a pressure signal, while the detected acceleration processor 78 detects the acceleration of the rescuer A's hand and generates an acceleration signal. Note that by performing the chest compressions on the rescuee B on a stable surface such as a floor, which substantially does not displace (vibrate) in the up-down direction, and detecting the acceleration, the acceleration of the rescuer A's hand during the chest compressions will be accurately detected. In addition, when detecting the acceleration, it is desirable to perform the chest compressions with the rescuee B supported on a hard surface which does not deform. This will avoid the detection of the acceleration due to deformation of the support surface, thereby accurately detecting the acceleration of the rescuer A's hand.

Then, the base compression depth calculator 80 calculates a base compression depth based on the signal of the acceleration detection value (acceleration signal), and generates a base compression depth signal. This base compression depth is calculated by performing second-order integration of the acceleration detection value. The base compression depth is calculated based on the acceleration accurately detected by, as described above, performing the chest compressions on the rescuee B on the stable support surface which does not displace or deform in the direction of chest compression (up-down direction). Generally, in many cases, the initial care of the rescue operation is performed on the rescuee B who collapsed on the stable surface such as a floor. Also, in the case where the rescuee B collapsed in an unstable place, the rescuee B is moved to a stable place before performing cardiopulmonary resuscitation. Thus, it is desirable to set the base compression depth based on the acceleration detection value detected by the acceleration sensor 64 at the initial stage of the rescue operation requiring cardiopulmonary resuscitation.

Besides, the pressure signal generated by the detected pressure processor 68 and the base compression depth signal generated by the base compression depth calculator 80 are transmitted to the correlation estimator 70 of the pressure detecting device 12, and the correlation estimator 70 calculates a correlation equation of the pressure detection value (electrostatic capacity value) detected by the detected pressure processor 68 and the base compression depth. The correlation equation of the pressure (electrostatic capacity) detection value (P(x)) and the chest compression depth (D(y)) is represented by, for example, D(y) = k • P(x). Here, the constant k is calculated to be k = D'(y)/P'(x) based on the base compression depth D'(y) and the electrostatic capacity detection value P'(x) when calculating the base compression depth.

The correlation estimator 70 may set the correlation equation based on the detection value with respect to a single chest compression. However, by setting the correlation equation based on the detection values with respect to a plurality of chest compressions, it is possible to estimate the relationship between the pressure detection value and the chest compression depth with a higher degree of accuracy. It is also acceptable to impart the function for resetting the correlation equation set by the correlation estimator 70 and redoing the detection by the acceleration sensor 64 as well as the setting of the correlation equation based on the detection value. In that case, the setting of the correlation equation may be automatically redone in accordance with the condition set in advance, for example, or alternatively, the reset of the correlation equation may be instructed manually by providing a reset operator such as a switch on the pressure detecting device 12 or a touch switch shown on the display 62 of the smart phone 14 so as to have the correlation estimator 70 execute the reset of the correlation equation.

After the correlation estimator 70 sets the correlation equation of the pressure detection value and the chest compression depth, in other words, the constant k of the above-mentioned correlation equation, the compression depth calculator 72 calculates the chest compression depth based on the pressure (electrostatic capacity) detection value and the correlation equation. By so doing, the individual variation of the amount of deformation of the rescuee B's chest in response to the compression (chest compression depth) is corrected by the correlation equation, thereby accurately obtaining the chest compression depth from the pressure detection value.

Moreover, by measuring the chest compression depth based on the pressure detection value, it is possible to avoid an adverse effect on the measurement accuracy due to deformation or displacement of the surface that supports the rescuee B. Specifically, in the case of moving with the rescuee B placed on the movement means such as a stretcher, an ambulance, or the like, the acceleration sensor 64 detects the acceleration due to vibration during the movement, the acceleration due to deformation of the stretcher, or the like. This may cause deterioration in accuracy of the chest compression depth measured based on the acceleration detection value. Here, the cardiopulmonary resuscitation support device 10 measures the chest compression depth based on the pressure detection value detected by the pressure sensor 16. This makes it possible to avoid the adverse effect on the measurement accuracy due to deformation or displacement of the surface that supports the rescuee B.

In this way, with the cardiopulmonary resuscitation support device 10, the chest compression depth is measured based on the pressure detection value, thereby avoiding the adverse effect on the measurement accuracy due to deformation or displacement of the surface that supports the rescuee B. Additionally, the correlation between the pressure detection value and the chest compression depth is set by utilizing the chest compression depth (base compression depth) based on the acceleration detection value accurately measured in the stable state before carrying the rescuee B. Therefore, it is possible to avoid errors in measurement results due to the ease in deformation of the chest that varies from one rescuee B to another.

Besides, the measured value of the chest compression depth obtained by the compression depth calculator 72 is transmitted as the chest compression depth signal to the display signal calculator 82 of the smart phone 14. The display signal calculator 82 generates a monitor display signal based on the measured value, and the detected information etc. is displayed on the display 62 in accordance with this monitor display signal (see FIG 6).

On the display monitor shown in FIG 6, a dummy illustration 86 equivalent to the rescuee B and a pressure distribution map 88 that shows distribution of the pressure acting on the chest of the dummy illustration 86 using different colors are displayed at the center part so as to overlap with each other. In addition, a compression depth graph 90 that shows time-dependent changes in the chest compression depth is displayed. Note that on the pressure distribution map 88, an arrow 92 indicates the direction to which the compression position should be moved.

Moreover, at the left end of the display 62, a simple evaluation area 94 is provided. The simple evaluation area 94 of the present embodiment displays simple indications such as a "○" for a good item and a "x" for a bad item, for 4 items including whether or not the maximum value of the chest compression depth is sufficiently large, whether or not the rhythm (tempo) of the chest compressions is correct, whether or not release of the chest is sufficient, and whether or not the chest compression time ratio (Duty Cycle, DC) is correct. Note that whether or not the release of the chest is sufficient can be determined by whether or not the minimum value of the chest compression depth is smaller than a threshold value.

Furthermore, at the right end of the display 62, an information display area 96 is provided. The information display area 96 of the present embodiment displays a call (communication) state to the emergency center 98 described later, a button to execute displaying a map of the AED location, and a fraction of chest compression time to the cardiac arrest time (Chest Compression Fraction, CCF). The button, which executes displaying a map of the AED location, displays a peripheral map of the current location and the place where the AED is located on the map by a touch operation on the display 62. The current location is specified by a GPS function of the GPS communicator 67 of the smart phone 14.

On the other hand, the connection detector 74 of the pressure detecting device 12 detects the connection of the pressure detecting device 12 to the smart phone 14 by short-range wireless communication such as Bluetooth (registered trademark), Wifi, infrared communication or the like, or by wire. Then, when the communication instructor 76 receives a connection detection signal from the connection detector 74, the communication instructor 76 determines that a use of the cardiopulmonary resuscitation support device 10 has started, and transmits a communication start signal to the communication processor 84 of the smart phone 14. It would also be possible for the communication instructor 76 to transmit the communication start signal based on a signal for detecting that the power supply of pressure detecting device 12 and the smart phone 14 turns to be on, for example. Also, for example, in the case where the pressure detecting device 12 is connected to the smart phone 14 by wire, even if the power supply of the pressure detecting device 12 is off, the connection may be detected so as to automatically start communication by the smart phone 14 described later.

The communication processor 84 that receives this communication start signal controls the wireless communicator 66 so as to automatically start communication with the emergency center 98 described later set in advance. That is, with the cardiopulmonary resuscitation support device 10, by the connection between the pressure detecting device 12 and the smart phone 14 being detected, the communication with the emergency center 98 will be automatically started by the wireless communicator 66. The communication mentioned herein may be, for example, a data communication to transmit and receive the detected information, electronic mails or the like, or may be a voice communication such as call, voice chat or the like. In the present embodiment, both the voice communication with an operator of the emergency center 98 and the data communication with a computer of the emergency center 98 will be automatically started.

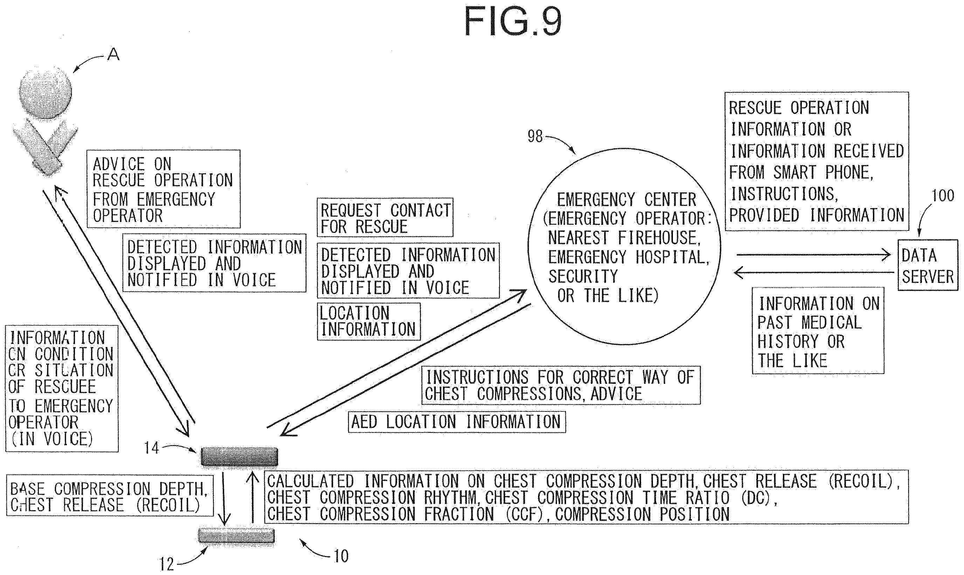

The cardiopulmonary resuscitation support device 10 of the present embodiment as described above is used, for example, during the emergency lifesaving by the rescuer A on the rescuee B, as shown in FIG. 9.

As a specific example, in the case where the rescuee B falls unconscious in a state of cardiac arrest or the like, the rescuer A at first launches an application related to the cardiopulmonary resuscitation support device 10 in the smart phone 14. By launching this application, the smart phone 14 and the pressure detecting device 12 are automatically connected with each other by short-range wireless communication such as Bluetooth (registered trademark), Wifi, infrared communication or the like. With the pressure detecting device 12 and the smart phone 14 wirelessly connected, the pressure detecting device 12 transmits the calculated information such as the chest compression depth, the chest release (recoil), the chest compression rhythm, the chest compression time ratio (DC), the chest compression fraction (CCF), the compression position, or the like, to the smart phone 14. On the other hand, the smart phone 14 transmits information about the base compression depth, the chest release (recoil), or the like to the pressure detecting device 12.

Moreover, when the smart phone 14 and the pressure detecting device 12 are connected with each other, the smart phone 14 automatically starts communication with the emergency center 98 (for example, the nearest firehouse, emergency hospital, security or the like). In the present embodiment, through the communication with the emergency center 98, a request contact for rescue such as a call for an ambulance is made automatically, as well as the location information (location information from which CPR information data is transmitted) obtained by the GPS communicator 67 of the smart phone 14 is transmitted to the emergency center 98. On the other hand, the emergency center 98 transmits information on the nearest AED location to the smart phone 14 based on the location information. If the application is accidentally launched, in order to prevent the communication with the emergency center 98 from automatically starting, it is acceptable to, for example, trigger the connection between the pressure detecting device 12 and the smart phone 14 by the power supply of the pressure detecting device 12 being turned on, or require the connection operation between the smart phone 14 and the pressure detecting device 12. Also, in the present embodiment, a peripheral map of the current location and the place where the AED is located on the map is displayed on the display 62 of the smart phone 14 based on the information on the AED location provided by the emergency center 98. However, the way of guidance to the AED location through the communication with the emergency center 98 is not limited to this, and it would also be possible to receive the guidance from an emergency operator by a call, for example. Besides, the information on the AED location may be stored in the smart phone 14 in advance.

In the case where the smart phone 14 and the pressure detecting device 12 are connected by wire, the data can be transmitted and received between the smart phone 14 and the pressure detecting device 12 through wired connection between the smart phone 14 and the pressure detecting device 12. Moreover, in the case where the smart phone 14 and the pressure detecting device 12 are wirelessly connected as well, automatic connection is not always required at the time of launch of the application, but for example, the application may be automatically launched when the wireless connection is completed.

Next, as depicted in FIGS. 1 through 3, the rescuer A overlaps the pressure detecting device 12 on the chest of the rescuee B laid on his or her back. The rescuer A arranges the pressure sensing part 40 of the pressure detecting device 12 on the rescuee B's chest so that the positioning line 30 of the pressure detecting device 12 is roughly aligned with the lateral center of the rescuee B. Besides, the pressure detecting device 12 is arranged so that the center marker 32 of the pressure detecting device 12 is roughly aligned with the approximate center of the chest compression position of the rescuee B.

Then, the rescuer A starts cardiopulmonary resuscitation using chest compressions. At this point, by the rescuer A performing the chest compressions with the smart phone 14 retained in his/her hand, the acceleration sensor 64 of the smart phone 14 detects the acceleration of the rescuer A's hand during the chest compressions.

Here, as depicted in FIGS. 1 and 2, the smart phone 14 is retained by the rescuer A's hand at a part away from the surface for compressing the rescuee B's chest (chest compression surface). Specifically, the chest compression surface of the rescuer A is defined by the palm and the finger cushions of the rescuer A that touch the pressure sensing part 40 of the pressure sensor 16 and compress the rescuee B's chest. Thus, the smart phone 14 is retained by, for example, the back of the rescuer A's hand which is away from the chest compression surface, or the opposite hand that overlaps the hand touching the pressure sensing part 40 of the pressure sensor 16.

Note that the smart phone 14 may be worn with a band, a tie, or the like on the rescuer A's hand or clothes covering the hand, or may be held by the rescuer A to be retained. Also, the smart phone 14 may be worn on the arm if the acceleration of the rescuer A's hand can be effectively detected during the chest compressions. However, in preferred practice, the smart phone 14 is retained at a position nearer to the hand than the elbow joint, and more preferably at a position beyond the wrist. That is, the rescuer A's hand that retains the smart phone 14 refers not only to the part beyond the wrist but to the overall upper limb including the arm beyond the shoulder.

Besides, the pressure sensor 16 of the pressure detecting device 12 detects the pressure acting on the rescuee B's chest due to the chest compressions. Then, the chest compression depth is calculated from the pressure detection value detected by the pressure sensor 16 based on the correlation equation of the pressure detection value and the chest compression depth. Note that it is acceptable as long as the smart phone 14 is retained by the rescuer A's hand during setting of the correlation equation of the pressure detection value and the chest compression depth based on the acceleration detection value. Thus, after the measurement of the chest compression depth based on the pressure detection value and the correlation equation has started, the smart phone 14 is not necessarily retained by the rescuer A's hand.

The information including the chest compression depth measured in this way is transmitted to the smart phone 14, and is provided to the rescuer A by being displayed on the display 62 (see FIG 6) or the like. By so doing, the rescuer A is able to grasp the points to be improved for the chest compressions being performed, and to perform the chest compressions more effectively.

Moreover, as illustrated in FIG 9, the rescuer A is able to perform the rescue operation while making contact with an operator of the emergency center 98 through a call or communication using the smart phone 14. Specifically, the rescuer A is able to provide the operator of the emergency center 98 with the information including the condition or situation of the rescuee B in voice, as well as to transmit the detected information of the chest compressions to the computer etc. of the emergency center 98 from the smart phone 14. On the other hand, the operator of the emergency center 98 is able to give advice on the rescue operation to the rescuer A in voice or by displaying on the display 62 based on the information transmitted from the smart phone 14, the information provided by the rescuer A, or the like.

Meanwhile, the information received by the emergency center 98 from the smart phone 14, the instruction and information provided by the emergency center 98 to the rescuer A, or the like is configured to be stored in a data server 100. Specifically, the information including where the rescue operation was performed, who performed the rescue operation, the symptoms of the rescuee B, the details of the rescue operation performed by the rescuer A, the details of the instruction on the rescue operation given by the emergency center 98 to the rescuer A, or the like is transmitted from the emergency center 98 to the data server 100 and stored therein to turn into big data. Then, using the information accumulated in the data server 100, it is possible to realize more effective study in the rescue operation, to provide the information on the medical history in the past of the rescuee B regarding another rescue operation, or the like.

Whereas the present embodiment exemplified the case where a single rescuer performs the rescue operation, in the case where a plurality of rescuers cooperate to perform the rescue operation as well, improvement in the lifesaving rates can be achieved by using the cardiopulmonary resuscitation support device 10. In preferred practice, for example, the detected information on the chest compressions as shown in FIG. 6 is displayed on the display 62 of each smart phone 14 of the plurality of rescuers. By so doing, it is possible for the rescuer performing the chest compressions to be advised by other rescuers based on the display monitor, or for the other rescuers to communicate with the emergency center 98, and the like. When the detected information is provided to the plurality of smart phones 14 in this way, for example, it may be acceptable to provide the information to all the smart phones 14 that exist within a prescribed distance area from the pressure detecting device 12 or the smart phone 14 of the main rescuer. Alternatively, the information may be selectively provided to the smart phones 14 selected on conditions that the smart phone 14 has read the two-dimensional code added to the pressure detecting device 12, or the application has been installed therein, or the like. However, it is desirable that the detected information on the chest compressions be provided only to the smart phones 14 positioned near the pressure detecting device 12, where the rescue operation is possible.

Moreover, in order to gather cooperators for the rescue operation, it would also be acceptable to provide means for notifying people in the periphery of the site of the rescue operation that the rescue operation is being performed. Specifically, the smart phone 14 of the rescuer A may automatically make communication for requesting rescue cooperation with the smart phones 14 that exist within a prescribed distance area, so as to request for the rescue cooperation to people in the periphery by visual notification means such as a monitor display, auditory notification means such as a sound by a speaker, and tactual notification means such as a vibration, or the like. Besides, the notification to the periphery may be made by a sound (voice) from a speaker provided to the pressure detecting device 12 or the smart phone 14, for example, or by an emission of a light, and the like.

An embodiment of the present invention has been described in detail above, but the present invention is not limited to those specific descriptions. For example, the pressure detecting device and the displacement detecting device need not necessarily be directly connected with each other. It would also be acceptable that the pressure detecting device and the displacement detecting device are separately connected to terminals such as a personal computer, a tablet, or the like, and the detected information is transmitted to the personal computer or the tablet.

Also, the specific shape or size of the pressure detecting device, the number of the pressure detection parts or the like, is not limited in particular. Similarly, the specific shape, size, or the like of the displacement detecting device is not limited in particular either. Besides, while the displacement detecting device preferably comprises the smart phone 14 exemplified in the preceding embodiment, it may comprise a mobile terminal exclusive to the cardiopulmonary resuscitation support device, for example. In addition, in the displacement detecting device, the wireless communicator 66 to communicate with the emergency center 98, the GPS communicator 67 to obtain the location information, or the like are not essential.

Moreover, the contents displayed on the display monitor of the displacement detecting device is appropriately variable, and the display monitor itself may be omitted. Note that the display monitor may be provided to the pressure detecting device, or may be provided to the personal computer or the tablet mentioned above.

Furthermore, the wireless communicator 66, the GPS communicator 67, the base compression depth calculator 80, or the like may be provided to the pressure detecting device 12. On the other hand, the connection detector 74, the communication instructor 76, the correlation estimator 70 or the like may also be provided to the displacement detecting device.

Besides, no particular limitation is imposed as to the detection method of the pressure sensor. For example, it is also possible to employ a resistance type pressure sensor for which an elastic part is formed of a conductive rubber made by mixing a conductive filler with a rubber material, and during a load input, the pressure is detected based on the change in electrical resistance of the elastic part due to elastic deformation of the elastic part. In addition, for example, an electromagnetic induction type pressure sensor may also be employed for which a substrate having a coil pattern and a conductive sheet are vertically positioned in opposition via an elastic part, and the pressure is detected based on the electromagnetic inductance effect due to approach of the conductive sheet to the substrate.

Additionally, the preceding embodiment described an example wherein the emergency center 98 provides the smart phone 14 of the rescuer A with the information on the AED location. However, the means for rapidly carrying the nearest AED to the rescue site is not necessarily limited to providing information to the smart phone 14. As a specific example, it would also be possible for the emergency center 98 to make contact with the AED location which is nearest to the rescue operation site for a request to carry the AED to the rescue operation site. Moreover, for example, it would also be possible to transmit a notification signal from the emergency center 98 to the AED location which is nearest to the rescue operation site, and there, notification to the periphery is started by a sound (voice) using a speaker, by an illumination of a light, or the like, so as to request people in the periphery to carry the AED to the rescue operation site.

In the preceding embodiment, the acceleration sensor 64 is exemplified as the displacement sensor. However, it is sufficient for the displacement sensor to be able to determine the displacement of the rescuer A's hand based on the detection results obtained by the sensor. For example, an atmospheric pressure sensor that detects the displacement in the vertical up-down direction based on the change in atmospheric pressure or the like may be employed as the displacement sensor.

## Claims

1. A cardiopulmonary resuscitation support device (10) for assisting a rescuer with cardiopulmonary resuscitation using chest compressions, comprising:
a pressure detecting device (12) provided with a pressure sensor (16) including a pressure sensing part (40) which is configured to be overlapped on a chest, the pressure sensing part (40) having a sheet form and being capable of flexural deformation in a flexible manner; and
a displacement detecting device (14) provided with a displacement sensor (64) to detect a displacement of a hand of the rescuer compressing the chest, the displacement detecting device (14) being configured to be retained by the hand of the rescuer at a part away from a chest compression surface.

2. The cardiopulmonary resuscitation support device (10) according to claim 1, further comprising:
a base compression depth calculator (80) to calculate a base compression depth for obtaining a chest compression depth based on the displacement detected by the displacement sensor (64); and
a correlation estimator (70) to set a correlation equation of a pressure detected by the pressure sensor (16) and the chest compression depth based on the pressure detected by the pressure sensor (16) and the base compression depth.

3. The cardiopulmonary resuscitation support device (10) according to claim 1 or 2, further comprising at least one display monitor (62) to display detected information.

4. The cardiopulmonary resuscitation support device (10) according to claim 3, wherein the at least one display monitor (62) comprises a plurality of display monitors (62) positioned near the pressure detecting device (12), and each of the display monitors (62) is configured to display the detected information.

5. The cardiopulmonary resuscitation support device (10) according to any one of claims 1-4, further comprising a wireless communicator (66) capable of communication with an emergency center (98).

6. The cardiopulmonary resuscitation support device (10) according to claim 5, further comprising a display monitor (62) to display a communication state by the wireless communicator (66).

7. The cardiopulmonary resuscitation support device (10) according to claim 5 or 6, further comprising a communication instructor (76) to automatically start the communication by the wireless communicator (66) when a use of at least one of the displacement detecting device (14) and the pressure detecting device (12) is detected.

8. The cardiopulmonary resuscitation support device (10) according to any one of claims 5-7, wherein the displacement detecting device (14) comprises a mobile phone (14), and the displacement detecting device (14) includes the wireless communicator (66).

9. The cardiopulmonary resuscitation support device (10) according to any one of claims 1-8, wherein
the pressure sensor (16) comprises a capacitance type pressure sensor having a structure in which a plurality of pressure detection parts (38) are arranged two dimensionally, each pressure detection part (38) comprising flexible electrodes (34a, 34b) placed in opposition and a flexible dielectric layer (20) having electric insulation properties disposed between the electrodes (34a, 34b), and
the pressure sensor (16) is configured to detect a pressure acting on the chest during the chest compressions based on an amount of change in electrostatic capacity of each pressure detection part (38) generated by a change in distance between the electrodes (34a, 34b) when the pressure acts due to the chest compressions.

10. The cardiopulmonary resuscitation support device (10) according to any one of claims 1-9, wherein the displacement sensor (64) of the displacement detecting device (14) comprises an acceleration sensor (64).
